# EUROPEAN PATENT SPECIFICATION

(11) **EP 2 178 476 B1**
(45) Date of publication and mention of the grant of the patent: **05.10.2016**
(21) Application number: 08797922.5
(22) Date of filing: 14.08.2008
(51) Int. Cl.: A61F 7/00, A61M 5/44, A61M 5/142, A61F 7/12

(54) **METHODS AND SYSTEMS FOR INDUCING THERAPEUTIC HYPOTHERMIA IN A PRE-HOSPITAL, FIELD, OR AMBULANCE SETTING**
VERFAHREN- UND SYSTEME ZUR HERBEIFÜHRUNG THERAPEUTISCHER HYPOTHERMIE IN EINER PRÄ-HOSPITAL-, FELD- ODER AMBULANZUMGEBUNG
PROCÉDÉS ET SYSTÈMES POUR INDUIRE UNE HYPOTHERMIE THÉRAPEUTIQUE EN CONDITIONS PRÉ-HOSPITALIÈRES, SUR LE TERRAIN OU EN AMBULANCE

(30) Priority: 14.08.2007 US 964630 P; 05.10.2007 US 978069 P; 14.12.2007 US 7642 P
(43) Date of publication of application: 28.04.2010
(73) Proprietor: ZOLL Circulation, Inc., San Jose, CA 95131 (US)
(72) Inventor: MAGERS, Michael, Encinitas, CA 92024 (US)
(74) Representative: D Young & Co LLP
(86) International application number: PCT/US2008/073211
(87) International publication number: WO 2009/023797

(56) References cited:
- EP-A1- 1 747 788
- WO-A1-97/05915
- WO-A1-97/39707
- WO-A1-98/31312
- WO-A2-01/43661
- US-A- 4 657 160
- US-A- 5 207 645
- US-A- 5 486 207
- US-A- 5 591 220
- US-A1- 2004 199 230
- US-A1- 2005 027 290
- US-A1- 2005 209 658
- US-A1- 2007 106 247
- US-B1- 6 406 458
- US-B1- 6 508 859
- US-B1- 6 508 859
- US-B1- 6 620 189

## Description

### STATEMENT OF RELATED APPLICATIONS

This application claims the benefit of U.S. Provisional Patent Application Serial No. 60/964,630, filed August 14, 2007, entitled "Pre-Hospital Cooling."

This application also claims the benefit of U.S. Provisional Patent Application Serial No. 60/978,069, filed October 5, 2007, entitled "Methods and Systems For Inducing Therapeutic Hypothermia In A Pre-Hospital, Field, or Ambulance Setting."

This application also claims the benefit of U.S. Provisional Patent Application Serial No. 61/007,642, filed December 14, 2007, entitled "Methods and Systems For Inducing Therapeutic Hypothermia In A Pre-Hospital, Field, or Ambulance Setting."

### BACKGROUND

The benefits of therapeutic hypothermia have been well-documented. One way of causing a therapeutic state of hypothermia in a patient is by the use of surface cooling techniques such as ice baths.

In another example, the US 5 591 220 A discloses a portable heating and cooling system that can circulate cooling fluid through a pad which is applied externally to body parts. A similar pad is described in the US 5 486 207 A.

The US 6 620 189 B1 discloses an apparatus for controlling the temperature of a patient or a body region thereof via a heat exchange catheter through which a heat exchange medium is circulated. Outside the body, the heat exchange medium circulates through a cassette that can be inserted into a portable control unit or alternatively into a larger control unit. Application of a thermal therapy catheter is also disclosed in the WO 97/39707 A1 and the WO 01/43661 A2.

The WO 98/31312 A1 discloses a system for infusing a fluid into a patient, said system comprising a housing in which an IV bag with the fluid can be placed. The housing further comprises a temperatures altering element for controlling the temperature of the fluid and a compressor for applying pressure to the IV bag for a delivery of the fluid. A similar device is disclosed in the EP 1 747 788 A1.

The US 6 508 859 B1 discloses a device for removing entrained gas or air from an infusion liquid. The WO 97/05915 A1 relates to a pressure infusion apparatus in which a pressure is applied on an infusion bag to press out infusion liquid.

Recently, intravascular techniques have gained a foothold due to their increased controllability and speed. However, both of these techniques are typically administered in a hospital or clinical setting due to their difficulty in field administration.

Nevertheless, physicians desire to initiate significant cooling in the field, e.g., enroute to the hospital or ER. Therapeutic hypothermia has been suggested to be induced in the field by direct venous or arterial infusion of chilled solutions, typically 0.9% saline (e.g., Lactated Ringer's Solution) which is available in most clinical settings. If this fluid is injected at a temperature near 0°C, the effective 'cooling power' applied to perfused tissue is directly proportional to body temperature and the rate of infusion. Increasing the mass flux of infusate will result in a greater rate of heat extraction from perfused tissue, but there is a limit to the rate and ultimate amount of fluids that may be safely infused. This requirement is made stricter by certain clinical conditions such as AMI (heart attack patients are not typically given large amounts of fluid in order to minimize stress to which the heart is subjected).

Therapeutic hypothermia has also been suggested to be induced by infusing a slush mixture. In particular, if the total volume of fluid which can be administered to AMI patients is limited, then raising the effective heat capacity of the infused fluid may allow effective application of therapeutic hypothermia. If the infused fluid were a slush, or a mixture of water ice and a saline solution chosen so that the bulk composition matches that of 0.9% saline, then in addition to the heat capacity of the liquid saline, the total heat absorbed during equilibration with body temperature would include the latent heat available in the infused ice. This technique increases the effective 'cooling power' available by infusion of chilled fluids. However, creating and maintaining a proper source and mixture of slush is complex.

In general, current techniques for administering such cooling tend to be non-standard and non-reproducible. Added to the above difficulties is that EMS care to cardiac arrest survivors tends to be very turbulent and hectic, and the availability of proper liquids, refrigeration capability, and delivery protocols is minimal.

### SUMMARY

Provided herein are methods, systems and apparatuses for use in delivering a cooled liquid to the vasculature of a subject in need thereof. As such, the methods, systems and apparatuses of this disclosure may be used, for example, to reduce the body temperature of a subject or to induce a therapeutic state of hypothermia in a subject. The methods, systems and apparatuses are of particular use in an emergency room or settings outside of a hospital. In some embodiments, the apparatuses are configured to fit and/or be used in an emergency vehicle (such as an ambulance, helicopter or other emergency medical services (EMS) vehicle). In some embodiments, the apparatuses or portions of the apparatuses are portable so that they can be used in the field and/or during transport of the subject. Accordingly, cool liquid can be provided to the subject as soon as possible and continuously until further treatment can be begin, if necessary.

In one embodiment, the invention provides an apparatus for use in delivering a cooled liquid from a liquid-containing package to the vasculature of a subject in need thereof, the apparatus comprising a portable unit adapted to contain at least one liquid-containing package and a fixed based unit, the portable unit being attachable and detachable to the fixed base unit. The fixed base unit includes a power supply system and a means for cooling a liquid-containing package when the package is contained by the portable unit. The apparatus is for use with a liquid-containing package that has a dispensing port for discharging the liquid therethrough and the portable unit or the package is adapted to receive a line for delivering the liquid to the subject.

In one embodiment, portable unit of the apparatus for use in delivering a cooled liquid from a liquid-containing package to the vasculature of a subject in need thereof includes a pressurizer adapted to be in pressure communication with the package to cause a set pressure to be incident in the package, wherein the liquid is caused to flow out of the package upon activation of a valve. In some embodiments, the portable unit includes a battery that powers the pressurizer. In some embodiments, the portable unit is adapted to contain two liquid-containing packages. In some embodiments, the liquid-containing package is a pressurizable package, such as a bag.

In some embodiments, the cooled liquid may be delivered to the subject without the use of a pressurizer. In such cases, the liquid may be delivered through a large bore needle and or in conjunction with a agent which facilitates liquid infusion in tissue.

Embodiments of the invention provide standard and reproducible systems for inducing an artificial and therapeutic state of hypothermia in a patient, especially in emergency or pre-hospital settings. In certain embodiments, cold biocompatible liquid is infused into a patient at a defined temperature and pressure. The cold liquid may be infused at a fairly rapid rate to quickly induce hypothermia. In embodiments employing IV bags, unlike many such systems, operation is independent of the effects of gravity, and may be carried any with bags in any orientation or elevation, such as in a rescue helicopter.

In one embodiment, cold liquid is forced out of a refrigerated bag, the pressure on the bag arising from air pressure on the bag, an infusor cuff, or any other pressurization technique. The cold liquid may be cooled in advance of the infusion or via a cold plate which forms a portion of the system. The cold plate may be separated from the cold liquid and pressurization system prior to use. In another embodiment, the cold liquid is provided from a flowing source.

In one aspect, a method for inducing a therapeutic state of hypothermia is described. Steps include accessing a blood vessel of a patient, such that an intravenous line is fluidically coupled at a distal end to the interior of the patient's blood vessel; attaching a source of cooled liquid to a proximal end of the intravenous line, wherein the temperature of the cooled liquid is between about 0°C and 10°C; and pressurizing the source of cooled liquid such that cooled liquid is forced into the patient's blood vessel at a pressure of between about 100-500mmHg +/- 20%.

Implementations may include one or more of the following. The pressurizing may be such that between 0.5 and 2 liters of cooled liquid are delivered to the patient in a period of between about 15 minutes and 1 hour, and/or such that the patient's core temperature is depressed between about 0.5°C and 2°C over a period of time of between about 15 and 45 minutes. The source may be a flexible bag, and the pressurizing may include pressurizing the flexible bag. For example, the pressurizing may be performed by increasing the air pressure adjacent the bag or by mechanically squeezing the bag or by attaching a pump to the bag. The source of cooled liquid may be pre-cooled, such as by storing the source in an environment below room temperature, such as a cooler. The flexible bag may be an IV bag, and the pressurizing may be performed by an infusor bag. Air may be prevented from entering the patient's blood vessel by placing an air in-line eliminator between the source of cooled liquid and the patient's blood vessel.

Advantages of certain embodiments of the invention may include one or more of the following. The system may be highly standard and reproducible, leading to predictable delivery of cold liquids. The system are convenient to administer, and allow cooling for therapeutic hypothermia to be initiated rapidly following, for example, a heart malady or other ischemic situation.

Other details, features, and advantages will be apparent from the description that follows, including the drawings and figures.

### BRIEF DESCRIPTION OF THE FIGURES

Fig. 1 shows a schematic diagram of a first embodiment of the system, employing a fixed source of liquid.
Fig. 2 shows a schematic diagram of a second embodiment of the system, employing a constantly-flowing source of liquid.
Fig. 3 shows an exemplary pressurizer.
Fig. 4 shows details of the control system.
Fig. 5 shows a first embodiment of a method related to an apparatus of the invention.
Fig. 6 shows a second embodiment of a method related to an apparatus of the invention.
Fig. 7 shows a third embodiment of the system, employing a fixed source of liquid.
Fig. 8 shows a system diagram which may be employed in the third embodiment of the invention.
Fig. 9 shows a third embodiment of a method related to an apparatus of the invention, which may employ the system of Figs. 7-8.

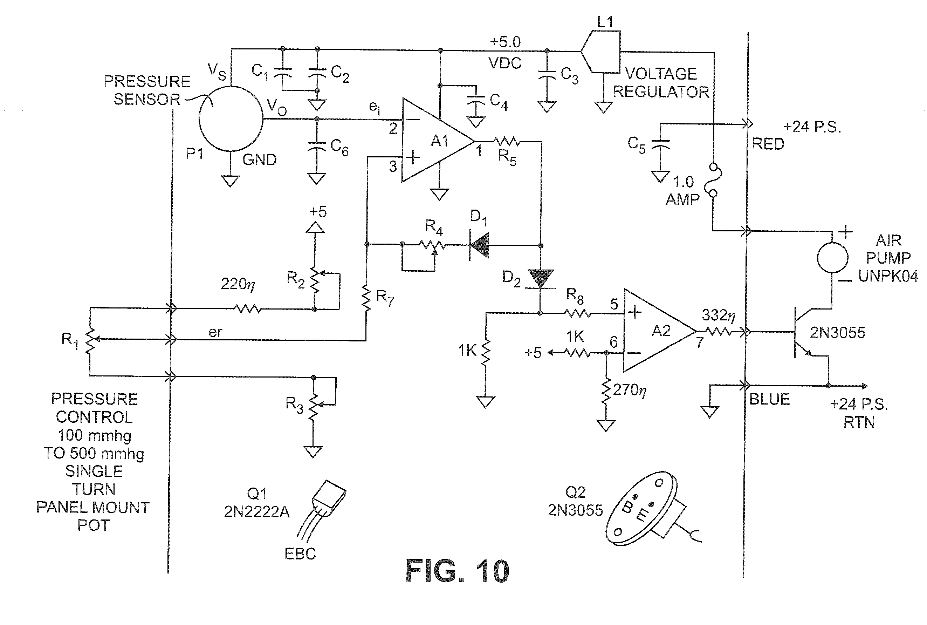

Fig. 10 shows an embodiment of a circuit diagram which may be employed in the invention for control of pressure.

### DETAILED DESCRIPTION

In this description, where the same component or same type of component is used in different embodiments, it retains the same reference numeral. Also, terms like "fluidically coupled" and "pressure communication" refer to the situation where an increase of pressure at one point is communicated to another point due to the general incompressibility of liquids. In particular, these terms are used to describe a situation between two points in a flow or in a standing fluid. If pressure is applied at one point, the second point will eventually feel effects of the pressure if the two points are in pressure communication. Any number of valves or elements may be disposed between the two points, and the two points may still be in pressure communication if the above test is met. For example, for a standing fluid in a pipe, any number of pipe fittings may be disposed between two pipes and, so long as an open path is maintained, points in the respective pipes may still be in pressure communication.

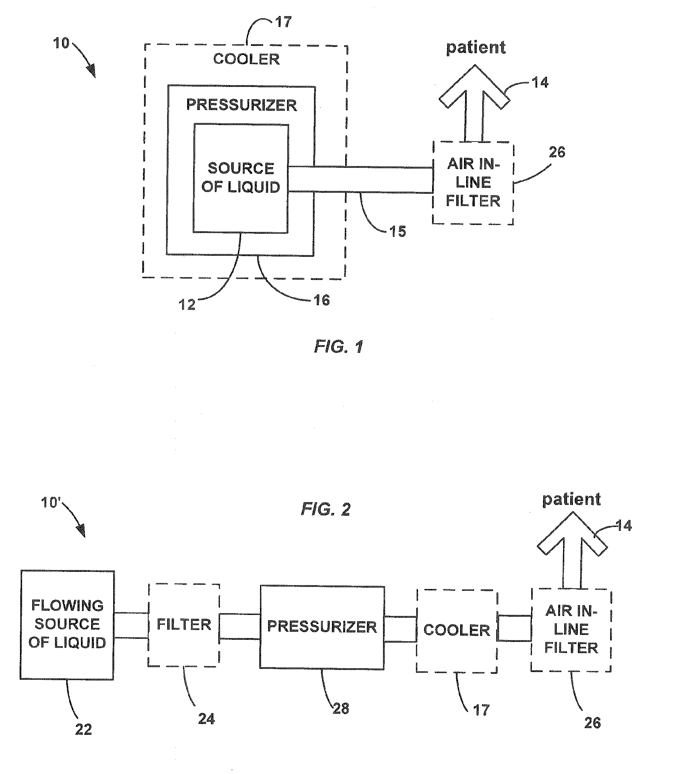

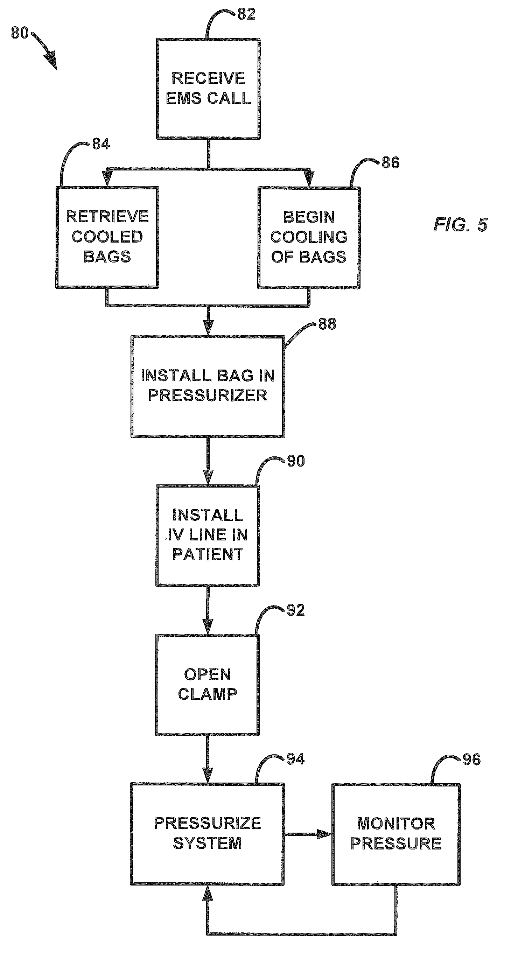

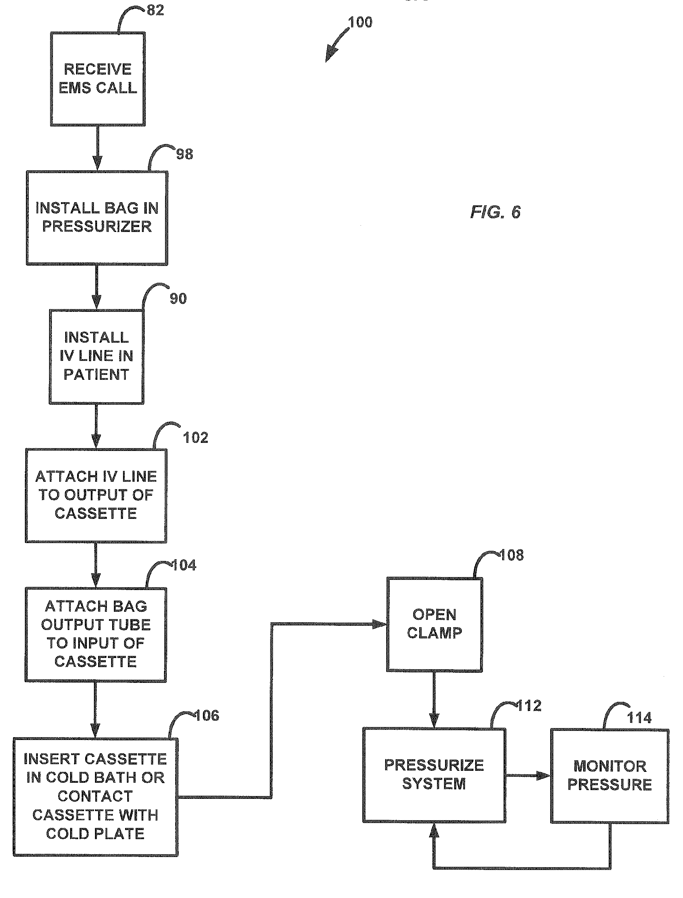

The system 10 and 10' (Figs. 1-4) and method 80 and 100 (Figs. 5 and 6) include the following components: a source of liquid, a catheter to introduce the liquid into a vein or artery of a patient (in most cases a vein), and a pressurization system. In some systems, such as where the source of liquid is not pre-cooled, the components further include a refrigeration system.

The source of liquid may be either contained, such as from an IV bag, or flowing. In the first case, as shown in Fig. 1, the source of liquid is shown as a bag 12. In this case, the pressurizer 16 pressurizes the source of liquid 12 and the same is transported into the patient via catheter 14, which may be an IV line.

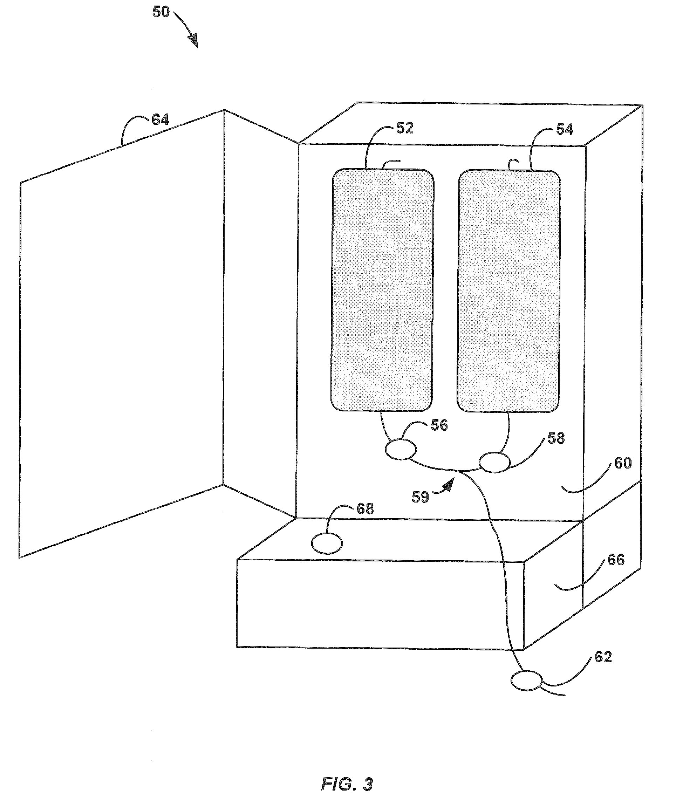

In Fig. 1, the pressurization system may include an air-tight chamber (see also Fig. 3) into which the source of liquid is placed. By pressurizing the chamber pneumatically, i.e., by introducing pressurized air into the air-tight chamber, the source of liquid may be compressed and the liquid within forced out of the bag via an outlet tube 15. The pressure within the air-tight chamber may be monitored and / or fed back to a controller to maintain constant pressurization and / or to eliminate overpressure situations. In an alternative embodiment, the pressurizing may occur via a pressure plate which directly contacts and exerts pressure on the source of liquid. In this embodiment, the pressure plate may be driven pneumatically or mechanically (e.g., including via a clutched driving mechanism or spring). In a further embodiment, the pressurizer may be a pump that forces the liquid into the catheter and thus into the patient; in this embodiment, suitable types of pumps may include roller pumps, peristaltic pumps, diaphragm pumps, vane pumps, gear pumps, and other pumps that can deliver on the order of 5-10 psi (above atmospheric pressure). In a system employing feedback to eliminate overpressure situations, the fed-back signal may be delivered to the pump controller rather than to a pneumatic or mechanical pressure plate controller. Various valves may be disposed at locations within the system and circulation set to stop or allow flow as necessary.

The liquid may be pre-cooled, in which case no refrigeration is necessary (e.g., the liquid bag may be stored in a cooler or refrigerator prior to use). If the liquid is not pre-cooled, then the pressurizer may be used in conjunction with a refrigeration system 17 to cause the liquid to be cooled prior to or contemporaneous with infusion. For example, where a pressurizer forces liquid from a bag, one wall of the pressurizer may be formed of the cooling unit of a refrigerator. To enhance heat transfer, the non-pre-cooled liquid may flow through a serpentine path (such as in a cassette) that is in thermal communication with the refrigeration system. The cassette may contact the wall of a thermoelectric generator (TEG) or may be situated within a cold water bath (which may in turn be cooled by, e.g., a TEG). Alternatively, the entire pressurizer may be subject to the cooling action of a refrigeration system. Any refrigeration system may be employed which results in cooling and temperature reduction of the liquid as well as to maintain the temperature of the cooling liquid at a target value.

If the source of liquid is constantly flowing, such as from a tap source and as shown in Fig. 2, the liquid may be from a source 22 that provides a constant flow of medical-grade and biosafe liquid. Alternatively, the liquid may be passed through a filter 24 so that the emerging liquid is safe for intravenous introduction (filters may in some cases be desired in the embodiment of Fig. 1 as well). In the embodiment of Fig. 2, the source may emerge with enough pressure such that no pressurizer is needed. Alternatively, the pressurizer 28 may be a pump. Suitable types of pumps include those listed above. A pump or other pressurizer, including those discussed above in connection with Fig. 1, may be employed in combination with the constantly-flowing source of liquid 22. If the liquid is flowed at a low temperature, no cooler is needed. Otherwise, cooler 17 may be employed to cool the liquid. The cooler 17 may be of a cassette or other type as described above.

Whether the liquid is pumped or pressurized for pneumatic or mechanical pressure, the liquid may be forced into the patient via the intravenous line 14. To overcome the blood pressure, the liquid pressure may be greater than the blood pressure. Suitable pressures may be between about 100-500mmHg +/- 20 %, more preferably 300mmHg +/- 10 %, such as 300mmHg (relative to ambient air pressure). Generally, the pressure has to be greater than that necessary to overcome the blood pressure in the blood vessel. In many cases, a maximum infusion pressure may be about 500mmHg. The temperature of the infused liquid may be between about 0°C and 10°C, more preferably 4°C to 5°C.

Whatever the pressure, the pressurization should be such as to be able to infuse at a flow rate of, e.g., 100 ml/minute. In particular, if the clinical need is for, e.g., 30 mg/kg (30 milligrams of cold liquid per kilogram of patient body mass) over 15-30 minutes, then a 70 kg patient would require approximately 2 liters infused over the course of 30 minutes.

In any embodiment, as an additional safety feature, an air in-line eliminator 26 may be employed to remove any air bubbles from the fluid prior to its introduction into the patient. In this way, the chance of air entering the patient's bloodstream is reduced.

In any embodiment, suitable liquids that may be infused include saline, lactated Ringer's solution, or any other such biosafe liquid. In addition, the device or method can be used to administer a variety of liquids including, but not limited to, saline solutions, blood, blood volume expanding fluids, drugs, solutes, nutrients and other physiologic fluids.

### EXAMPLE

As noted above, in one system, it is desired to infuse up to 2 liters of cold saline in 30 minutes. The exemplary system of Fig. 3 provides one system to perform this infusion. The system 50 includes two 1000 ml IV bags 52 and 54 of appropriate liquid, such as saline. Clamps 56 and 58 may arrest liquid flow prior to the desired infusion, and the IV lines may conveniently meet at a Y-connector 59, so that switching between bags is simplified. The bags rest against a cold plate 60, which may be the cold plate of a TEG. An air in-line eliminator 62, e.g., a bubble trap, may be situated between the bag and the patient to prevent infusion of dangerous air bubbles. A door 64 may close over the bags, and the door 64 may be clear so that liquid levels may be observed. The door 64 provides an air-tight enclosure, so that when air is forced into the chamber with the IV bags, the pressurized air efficiently compresses the bags, and does not leak to the exterior. The air pump may be disposed in section 66 of the system and may emerge via inlet 68. One pressure that may be employed is 0,40 bar (5.8 psi) within the chamber, or 300 mmHg.

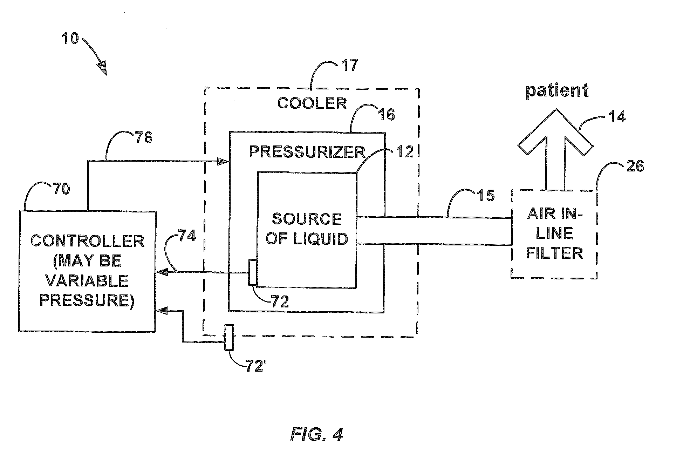

The system and method may employ a pressure feedback system to ensure that the infusion pressure does not reach beyond the desired pressure or to deleterious levels or that the infusion pressure maintains the desired liquid flow rate. In more detail, to ensure that the pressure is maintained at an appropriate level and does not reach unsafe levels, a pressure monitor may be employed that generates a signal that is fed back to a pressure controller. The pressure monitor may have a sensor that senses the pressure applied to a bag or other source of liquid, where that sensed pressure can be correlated with the pressure of the liquid infused. Alternatively, the pressure sensor may directly monitor the pressure of the liquid infused, such as via an in-line pressure sensor. Both such sensors may also be employed simultaneously in some systems. In any case, the pressure controller need not be a separate unit; rather, it may form a portion of a pump or a controller or the pressurization system. In particular, as shown in Fig. 4, a pressure sensor 72 may send a signal corresponding to the pressure via signal path 74 to a controller 70, which in turn sends a signal via path 76 to the pressurizer, to raise or lower the pressure as desired. In pump systems, the speed of the pump is controlled. The controller may be contained within the pressurizer, or may be a separate unit. Similarly, a temperature sensor 72' may send a signal corresponding to the temperature via a signal path to the controller 70, which may then be used to control the pressure, the speed of the pump, the temperature of the fluid (if modifiable), or other parameters.

Embodiments of a method related to the invention are shown in Fig. 5 and Fig. 6. In Fig. 5, a method is employed which may advantageously use the system of Fig. 3 (as well as other such systems). In Fig. 6, a cassette cooler system is used to cool the liquid *en route* to the patient.

In Fig. 5, an EMS call is received (step 82). The EMS service provider may retrieve a pre-cooled bag (or other source of liquid) (step 84), or may begin to cool a non-pre-cooled bag (step 86) by placing the same in a refrigeration unit. If the bag is non-pre-cooled, the EMS service provider may perform the step during the transport period to the patient. If the system is employed in an ER setting, the bags may typically be pre-cooled, although this is not strictly required. The bag is placed in a pressurizer (step 88). An IV line is installed in the patient (step 90). A clamp or other valve may be opened (step 92), permitting liquid infusion. The system is pressurized (step 94), the pressure is monitored (step 96), and the sensed pressure is employed as a control for the pressure administration.

In Fig. 6, again an EMS call is received (step 82). Once on-site, or before, the EMS service provider may install a non-pre-cooled bag in the pressurizer (step 98). A non-pre-cooled bag may also be installed before-hand, if desired. The EMS service provider may operate any employed refrigeration unit so that the system is ready when infusion liquid is pumped through the same. An IV line is installed in the patient (step 90), and the proximal end of the IV line is attached to the outlet of a cassette (step 102). The outlet tube of the bag or other source of liquid is attached to the inlet of the cassette (step 104). The cassette is inserted in a cold bath or is contacted with a cold plate or is otherwise cooled (step 106). A clamp or other valve may be opened (step 108), permitting liquid infusion. The system is pressurized (step 112), the pressure is monitored (step 114), and the sensed pressure is employed as a control for the pressure administration.

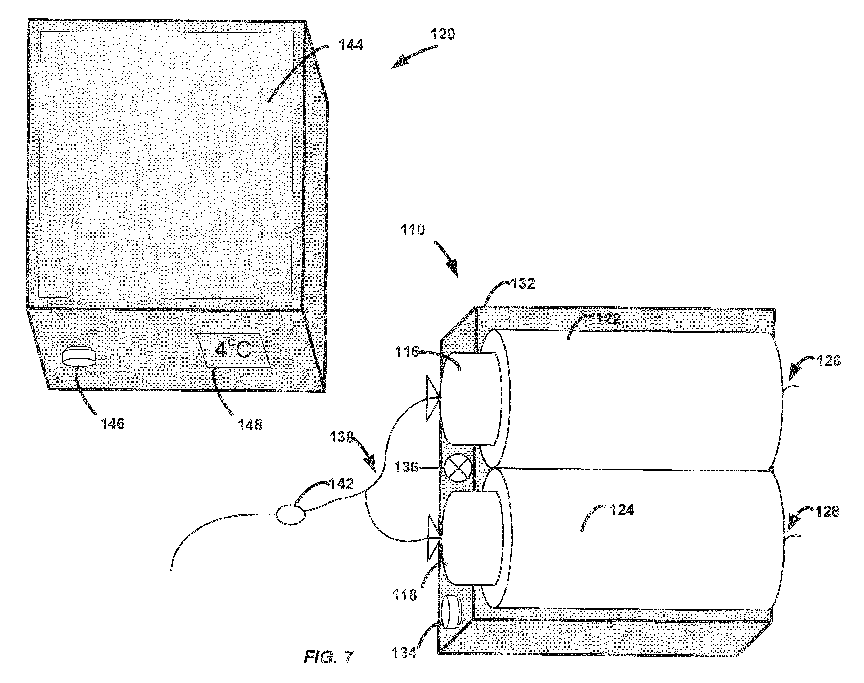

In another embodiment, as shown in Fig. 7, a system is employed in which a liquid-containing package, such as a pressurizable liquid-containing (e.g., IV) bag, in a portable unit, may be removed from a cold environment or from contact with a cold plate in a fixed base unit and hung on an IV pole for ease of administration to a patient, in the field or in any other emergency setting.

In particular, the system may be employed in an ambulance setting. To secure to an ambulance shelf, the fixed base unit may have a width of less than about 81,3 cm (32"), a height (when combined with the portable unit) of less than about 40,6 cm (16"), and a depth of less than about 50,8 cm (20"). As such cabinets are generally several feet in front of the patient's head, any connections from the system to the patient may generally travel in front of the ambulance's side door to the patient. This travel is generally inconvenient, and thus the embodiment of Fig. 7 allows removal of a disposable / reusable portable unit from a fixed base unit so that the portable unit may be placed adjacent the patient at the time of infusion.

This system includes a fixed base unit 120 and a portable unit 110, the portable unit being attachable and detachable from the fixed base unit. The fixed base unit includes a cold plate 144 for cooling (or heating, if necessary) a liquid-containing bag such as an IV bag. The fixed base unit generally operates on wall-provided power, as may be available in an ambulance or a hospital, and an on/off switch 146 control delivery of this power to the cold plate 144. The system may also operate using the 12 volt battery system in an ambulance. To secure the fixed base unit against movement, the same may be locked down to the appropriate shelf in the ambulance. A temperature display 148 shows the temperature of the cold plate by virtue of a temperature sensor disposed in thermal communication with the same.

The portable unit 110 (which includes some disposable components and some reusable components, "disposable/reusable") includes one and preferably two 1-liter IV bags 116 and 118 which may be placed and secured within pressurizable bags, such as infusor bags 122 and 124, respectively, which are in turn secured against a conductive plate 132 so that heat from the IV bags may be removed when the conductive plate 132 is placed atop the cold plate 144. In this way, the temperature of the IV bags is lowered as the liquid-containing bags are in thermal communication with the cold plate.

It is noted that while two 1-liter IV bags are discussed here, any number of such bags and infusor bags may be employed, according to the dictates of the user. Moreover, multiple IV bags may be placed within one infusor bag. Additionally, the liquid-containing bags need not be placed within the pressurizable bags; rather, any adjacent arrangement that allows the pressurizable bag to impart pressure on the liquid-containing bag may be employed. Finally, if sufficient thermal communication exists between the liquid-containing bags and the cold plate (often through the pressurizable bag), the conductive plate may be made smaller or eliminated.

When in preparation for use, the disposable / reusable portable unit 110 is placed (and optionally secured) to the fixed base unit 120, and the fixed base unit 120 is operated so as to cool the IV bags. When being readied for use with a patient, the disposable / reusable portable unit 110 is removed from the fixed base unit 120 and hung on an IV pole or other such attachment via IV tabs 126 and 128. For example, the disposable / reusable portable unit 110 may be hung from a hangar on the ceiling of the ambulance.

In some embodiments, the portable unit system generally includes the conductive plate, the infusor bag or bags, the IV bag or bags, as well as a pump, a pump battery, and monitoring circuitry for the pressurization. As noted above, the conductive plate may be omitted in arrangements where adequate thermal contact may be achieved directly between the cold plate and the combination of the IV bag and infusor bag. The pressurization may be via an air pump that is powered by a battery that forms part of the portable unit, and the battery may be recharged automatically upon contact with the fixed base unit. That is, the fixed base unit can recharge the pump battery when the portable unit is placed thereon. As discussed below, the pump may be controlled by appropriate monitoring circuitry.

The pump may also be operated by other techniques, include those involving gas cylinders or springs. Some other techniques, such as the use of pressurized gas, may eliminate the need for a pump: in this system, a controlled valve release air pressure that in turn pressurizes the liquid-containing bag.

The infusor bags 122 and 124 are pressurized to force fluid out of the IV bags at a set or determinable pressure. Some suitable pressure infusors that may be used are available from CasMed, Inc., Smiths Medical (and their Medex® unit), Cardinal Health (and their division Alaris® Medical Systems), Nellcor Puritan Bennett LLC (a division of Tyco Healthcare), and Mallinckrodt, Inc. (a division of Tyco Healthcare). Partial control of the flow of the fluid may be via a valve 142. The valve 142 is shown downstream of the y-connector 138, but similar valves may be disposed upstream of the y-connector 138 as well. A manometer may be included in the system, and the sensor for the same may be in one or more fluid lines from the IV bags 116 and 118, although in general the sensor may be located in any location that allows the same to measure the pressure of the liquid entering the patient. A pressure-setting knob 134 may be disposed on the system, as well as a pressure display 136. Various software or hardware or firmware may be employed to set a threshold pressure level, or maintain a specified pressure or flow rate, as well as to turn off the system if the threshold is exceeded. Alternatively, a physician may visually monitor display 136 for the in-line pressure, and control the same manually.

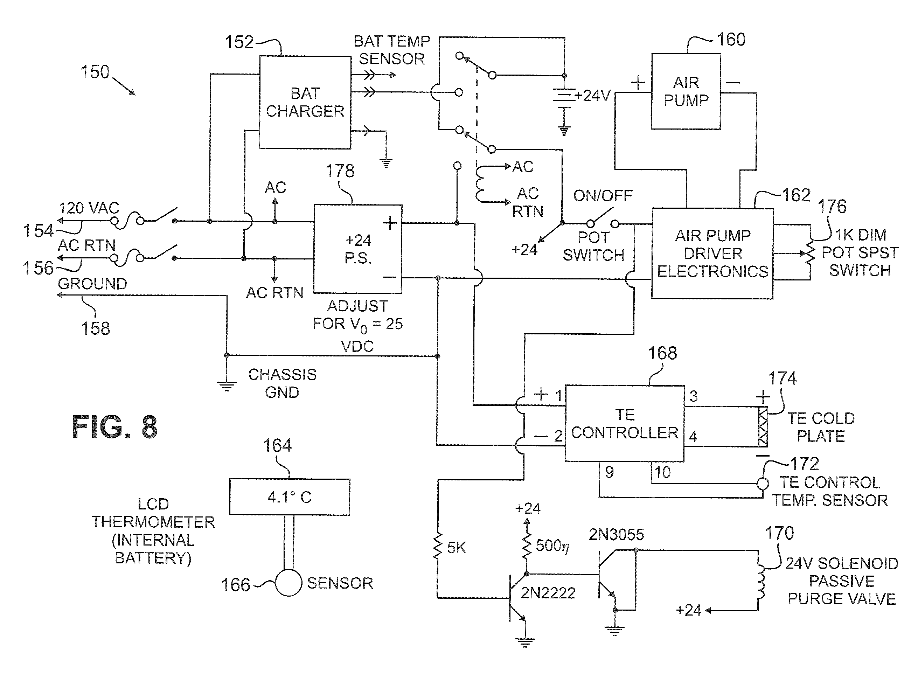

Fig. 8 shows a diagram of an exemplary circuit 150 that may be employed in the system of Fig. 7. AC wall power is shown with a hot line 154, return 156, and ground 158 feed into a power supply 178. The wall power also powers a battery charger 152. The power supply 178 further powers (through a transformer) air pump driver electronics 162, which are controlled by a switch 176. The power supply in addition powers the thermoelectric controller 168 which controls the thermoelectric cold plate 174. Feedback for the thermoelectric cold plate 174 is provided by a thermoelectric control temperature sensor 172. A solenoid pressure purge valve 170 is also provided to guard against overpressure situations as well as to purge or to "bleed off" pressure if necessary. A temperature sensor 166 provides a temperature display 164. One of ordinary skill in the art will recognize that numerous other circuit arrangements may also be employed.

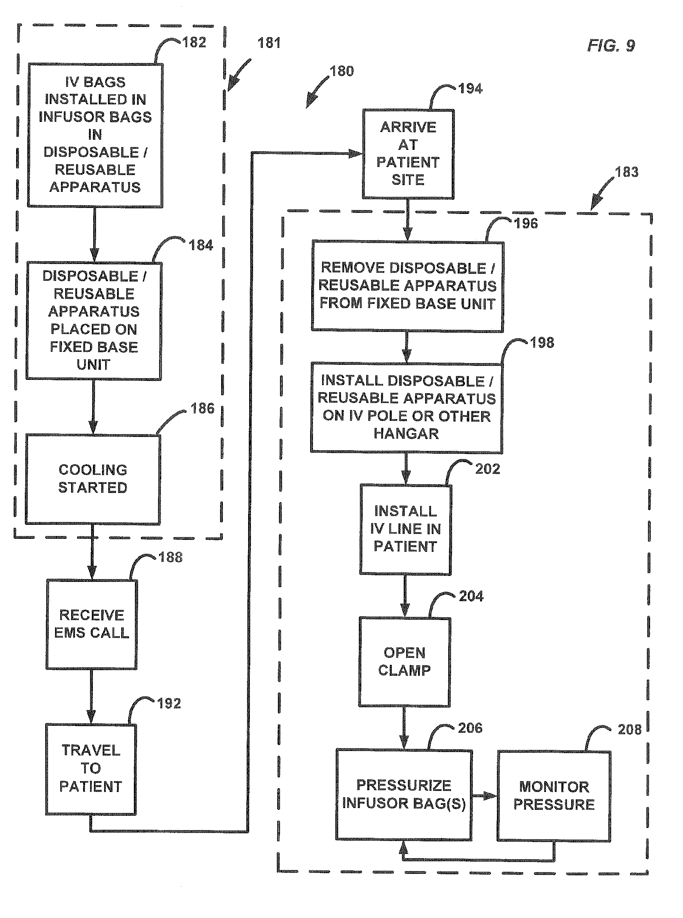

Fig. 9 shows an exemplary method 180 that may employ the system of Fig. 7. A submethod 181 is shown that is used to prepare the system for use, and a submethod 183 is shown to infuse cold fluid into a patient to induce a therapeutic state of hypothermia .

The method begins with installing one or more IV bags in a corresponding number of infusor bags (step 182). It is understood that various arrangements may be employed with specially-designed infusor bags such that more than one IV bag may be installed per infusor bag. The infusor bags may then be secured onto the conductive plate 132, or in some embodiments the infusor bags may be permanently secured thereon. If no conductive plate is employed, the pressurizable bags may be secured onto the housing containing the pump, pump battery, and monitoring circuitry. The monitoring circuit may also have a circuit arranged to monitor the temperature of the liquid in the liquid-containing bags.

The disposable / reusable portable unit 110 with the installed IV bags is then placed on the fixed base unit 120 (step 184). The fixed base unit 120 is operated so as to cool the bags (step 186). Power for the fixed base unit may be by way of a wall outlet in for example a hospital, an outlet in an emergency vehicle, for example an ambulance, which is powered by the vehicle battery, or an outlet in an emergency vehicle that is powered by an external power hook-up connected to the vehicle. Once the IV bag or bags are cooled to a suitable temperature, e.g., 4°C, the system is ready for use.

An EMS call may be received (step 188), and the system may travel to the patient (step 192). Of course, it is understood the system may be employed elsewhere, such as in a hospital or clinic setting. In any case, prior to and/or during any transit time to the patient, the fixed base unit 120 may operate to cool the bags within the disposable / reusable portable unit 110 so that their temperature is minimized by the time the system arrives at the patient (step 194) and infusion of cooled fluids is begun.

A first step in the method of treatment is to remove the disposable / reusable portable unit 110 from the fixed base unit 120 (step 196). The disposable / reusable portable unit 110 may then be installed on an IV pole or any other such device (step 198). As in embodiments above, an IV line may be installed in the patient (step 202). If a clamp or other valve mechanism has been employed on the administration or introduction set, the same is opened (step 204). The pressure is set on the disposable / reusable portable unit 110, and the infusor bags are appropriately and automatically pressurized (step 206) such that the pressure in the line achieves the set pressure. The pressure is monitored (step 208) and fed back to the system so that the set pressure is maintained to an appropriate degree of tolerance. The pressure may be set to the values described above.

As noted above, in any embodiment, the pressurization may occur via mechanical or pneumatic bag compression, via a separate pump, via an infusor cuff surrounding the source of liquid as described above, or via any other technique for forcing a liquid into a pressurized environment.

It should be noted that if packages, bags or other sources of liquid are only partially pre-cooled, the same may in addition undergo the refrigeration or other cooling steps noted above. The above steps are not intended to be mutually exclusive. Suitable refrigeration units may be available from, e.g., Engel USA, Sawafuji Electric Co. Ltd. (Japan). Suitable TEG systems are available from, e.g., TE Technology Inc. (Traverse City, MI).

Generally no anti-shivering drugs are required when the patients are comatose and no shivering response is activated. However, in the case where a shivering response is activated, anti-shivering drugs may be administered, such as meperidine, Demerol®, nefopam, buspirone, fentanyl, BuSpar, or any other anti-shivering drug.

The invention has been described with respect to a number of embodiments. One of ordinary skill in the art will recognize, however, that variations may be made within the scope and spirit of the invention. For example, while the invention has been generally described above in the context of an ambulance setting, the system and method may be used in an ER setting or in a hospital setting or in the field at the location of an injured individual (including e.g., nursing homes, military/battlefield sites). For example, in an ambulance setting, the system and method may be employed during a 20-30 minute ambulance ride to a treatment facility. Accordingly, the system described herein includes apparatus which are portable and powered by an AC and/or DC power source, including standard batteries or rechargeable batteries. Embodiments of the invention may be advantageously employed as part of a "crash cart" in an ambulance or hospital setting. Embodiments of the invention may be particularly useful in the treatment of cardiac arrest, stroke, traumatic injuries, unanticipated hyperthermia or hypothermia, and other such maladies. The system and method have been described primarily in the context of thermoelectric refrigerators, but conventional refrigerators and even non-refrigerated (but initially cooled, such as via ice packs) coolers may be employed. The system can be used for warming as well as cooling. In this case, the liquid should be delivered at a temperature greater than that of the body temperature, but usually less than about 42°C.

Accordingly, the invention is to be limited only by the claims appended hereto.

## Claims

1. An apparatus for use in delivering a cooled liquid from a liquid-containing package (116, 118) to the vasculature of a subject in need thereof, comprising:
a. a portable unit (110) adapted to contain at least one liquid-containing package (116, 118) comprising a dispensing port for discharging the liquid therethrough, wherein the portable unit (110) or the liquid containing package (116, 118) is adapted to receive a line for delivering the liquid to the subject,
wherein the portable unit (110) comprises a pressurizer (122, 124) adapted to be in pressure communication with the package (116, 118) to cause a set pressure to be incident in the package, wherein the liquid is caused to flow out of the package upon activation of a valve (142); and
b. a fixed base unit (120), including:
i. a power supply system; and
ii. a means (144) for cooling the liquid-containing package (116, 118) when contained by the portable unit (110);
the portable unit (110) being attachable and detachable to the fixed base unit (120).

2. The apparatus of claim 1, wherein the portable unit (110) further comprises a sensor to sense the pressure of the pressurizer (122, 124) or incident in the package (116, 118), and a controller to control the pressurizer (122, 124); the controller having an input from the sensor, wherein the controller operates the pressurizer (122, 124) to maintain the set pressure.

3. The apparatus of claim 1, wherein the portable unit (110) further comprises a battery that powers the pressurizer (122, 124).

4. The apparatus of claim 3, wherein the battery is rechargeable and the fixed base unit (120) further comprises a charger for the battery.

5. The apparatus of claim 1, wherein the pressurizer is a pressurizable bag (122, 124) configured to store the package (116, 118).

6. The apparatus of claim 1, wherein the pressurizer is a pressurizable infusion cuff.

7. The apparatus of claim 1, wherein the pressurizer acts via pneumatic compression of the package (116, 118).

8. The apparatus of claim 1, wherein the pressurizer (122, 124) acts via mechanical compression of the package (116, 118).

9. The apparatus of claim 1, wherein the pressurizer is a pump.

10. The apparatus of claim 1, wherein the portable unit (110) is adapted to contain two liquid-containing packages (116, 118).

11. The apparatus of claim 1, wherein the portable unit (110) is adapted to contain two liquid-containing packages (116, 118) and the pressurizer (122, 124) is adapted to be in pressure communication with both packages (116, 118).

12. The apparatus of claim 1, wherein the portable unit (110) is adapted to contain two liquid-containing packages (116, 118), and comprises two pressurizers (122, 124), each pressurizer (122, 124) adapted to be in pressure communication with a separate package (116, 118).

13. The apparatus of claim 1, further comprising the line for delivering the liquid to the subject.

14. The apparatus of claim 13, wherein the line further comprises an air in-line eliminator.

15. The apparatus of claim 1, wherein the cooling means (144) is configured to cool the liquid in the liquid-containing package (116, 118) to a temperature of between about 0° C and 10° C.

16. The apparatus of claim 1, wherein the cooling means includes a cold plate (144).

17. The apparatus of claim 1, wherein the fixed base unit (120) further comprises a temperature sensor and a controller to monitor and to control the temperature of the cooling means.

## Patentansprüche

1. Vorrichtung zur Verwendung beim Zuführen einer gekühlten Flüssigkeit von einem flüssigkeitsenthaltenden Behälter (116, 118) zu einem die Flüssigkeit benötigenden Gefäßsystem, wobei die Vorrichtung Folgendes umfasst:
a. eine tragbare Einheit (110), die geeignet ist, mindestens einen flüssigkeitsenthaltenden Behälter (116, 118) zu enthalten, der einen Ausgabeanschluss umfasst, durch den die Flüssigkeit abgegeben wird,
wobei die tragbare Einheit (110) oder der flüssigkeitsenthaltende Behälter (116, 118) geeignet ist, eine Leitung für das Zuführen der Flüssigkeit an den Zielgegenstand aufzunehmen,
wobei die tragbare Einheit (110) einen Druckhalter (122, 124) umfasst, der geeignet ist, in einer Druckverbindung mit dem Behälter (116, 118) zu stehen, um zu veranlassen, dass ein eingestellter Druck in den Behälter geleitet wird, wobei die Flüssigkeit veranlasst wird, nach der Aktivierung eines Ventils (142) aus dem Behälter zu fließen; und
b. eine feste Basiseinheit (120), die Folgendes umfasst:
i. ein Stromversorgungssystem; und
ii. ein Element (144) zum Kühlen des flüssigkeitsenthaltenenden Behälters (116, 118), wenn er in der tragbaren Einheit (110) enthalten ist;
wobei die tragbare Einheit (110) an die feste Einheit (120) anfügbar und davon abnehmbar ist.

2. Vorrichtung nach Anspruch 1, wobei die tragbare Einheit (110) außerdem einen Sensor umfasst, um den Druck des Druckhalters (122, 124) oder den in den Behälter (116, 118) geleiteten Druck zu erfassen, und eine Steuereinheit umfasst, um den Druckhalter (122, 124) zu steuern; wobei die Steuereinheit einen Eingang von dem Sensor aufweist, wobei die Steuereinheit den Druckhalter (122, 124) betreibt, um den eingestellten Druck beizubehalten.

3. Vorrichtung nach Anspruch 1, wobei die tragbare Einheit (110) außerdem eine Batterie umfasst, die den Druckhalter (122, 124) mit Strom versorgt.

4. Vorrichtung nach Anspruch 3, wobei die Batterie wiederaufladbar ist und die feste Basiseinheit (120) außerdem ein Ladegerät für die Batterie umfasst.

5. Vorrichtung nach Anspruch 1, wobei der Druckhalter ein mit Druck beaufschlagbarer Beutel (122, 124) ist, der konfiguriert ist, den Behälter (116, 118) zu speichern.

6. Vorrichtung nach Anspruch 1, wobei der Druckhalter eine mit Druck beaufschlagbare Infusionsmanschette ist.

7. Vorrichtung nach Anspruch 1, wobei der Druckhalter über eine druckluftbetätigte Komprimierung des Behälters (116, 118) funktioniert.

8. Vorrichtung nach Anspruch 1, wobei der Druckhalter (122, 124) über eine mechanische Komprimierung des Behälters (116, 118) funktioniert.

9. Vorrichtung nach Anspruch 1, wobei der Druckhalter eine Pumpe ist.

10. Vorrichtung nach Anspruch 1, wobei die tragbare Einheit (110) geeignet ist, zwei flüssigkeitsenthaltende Behälter (116, 118) zu enthalten.

11. Vorrichtung nach Anspruch 1, wobei die tragbare Einheit (110) geeignet ist, zwei flüssigkeitsenthaltende Behälter (116, 118) zu enthalten und der Druckhalter (122, 124) geeignet ist, mit beiden Behältern (116, 118) in einer Druckverbindung zu stehen.

12. Vorrichtung nach Anspruch 1, wobei die tragbare Einheit (110) geeignet ist, zwei flüssigkeitsenthaltende Behälter (116, 118) zu enthalten und zwei Druckhalter (122, 124) umfasst, wobei jeder Druckhalter (122, 124) geeignet ist, mit einem separaten Behälter (116, 118) in einer Druckverbindung zu stehen.

13. Vorrichtung nach Anspruch 1, die außerdem die Leitung für das Zuführen der Flüssigkeit an den Zielgegenstand umfasst.

14. Vorrichtung nach Anspruch 13, wobei die Leitung außerdem einen Leitungsentlüfter umfasst.

15. Vorrichtung nach Anspruch 1, wobei das Kühlelement (144) konfiguriert ist, die Flüssigkeit in dem flüssigkeitsenthaltenden Behälter (116, 118) auf eine Temperatur zwischen ungefähr 0 °C und 10 °C abzukühlen.

16. Vorrichtung nach Anspruch 1, wobei das Kühlelement eine Kälteplatte (144) umfasst.

17. Vorrichtung nach Anspruch 1, wobei die feste Basiseinheit (120) außerdem einen Temperatursensor und eine Steuereinheit umfasst, um die Temperatur des Kühlelements zu überwachen und zu steuern.

## Revendications

1. Appareil destiné à être utilisé pour administrer un liquide refroidi à partir d'un emballage (116, 118) contenant du liquide au système vasculaire d'un sujet qui en a besoin, comportant :
a. une unité portable (110) prévue pour contenir au moins un emballage (116, 118) contenant du liquide comportant un orifice de distribution servant à refouler le liquide à travers celui-ci, l'unité portable (110) ou l'emballage (116, 118) contenant du liquide étant prévus pour recevoir une conduite servant à administrer le liquide au sujet,
l'unité portable (110) comportant un pressuriseur (122, 124) prévu pour être en communication de pression avec l'emballage (116, 118) pour faire en sorte qu'une pression spécifiée règne dans l'emballage, le liquide étant amené à s'écouler hors de l'emballage suite à l'activation d'une vanne (142) ; et
b. une unité (120) de base fixe, comprenant :
i. un système d'alimentation électrique ; et
ii. un moyen (144) servant à refroidir l'emballage (116, 118) contenant du liquide lorsqu'il est contenu par l'unité portable (110) ;
l'unité portable (110) pouvant être rattachée à et détachée de l'unité (120) de base fixe.

2. Appareil selon la revendication 1, l'unité portable (110) comportant en outre un capteur servant à détecter la pression du pressuriseur (122, 124) ou régnant dans l'emballage (116, 118), et une commande servant à commander le pressuriseur (122, 124) ; la commande étant dotée d'une entrée en provenance du capteur, la commande faisant fonctionner le pressuriseur (122, 124) pour maintenir la pression spécifiée.

3. Appareil selon la revendication 1, l'unité portable (110) comportant en outre une batterie qui alimente le pressuriseur (122, 124).

4. Appareil selon la revendication 3, la batterie étant rechargeable et l'unité (120) de base fixe comportant en outre un chargeur pour la batterie.

5. Appareil selon la revendication 1, le pressuriseur étant un sac (122, 124) apte à être pressurisé, configuré pour stocker l'emballage (116, 118).

6. Appareil selon la revendication 1, le pressuriseur étant un manchon de perfusion apte à être pressurisé.

7. Appareil selon la revendication 1, le pressuriseur agissant par compression pneumatique de l'emballage (116, 118).

8. Appareil selon la revendication 1, le pressuriseur (122, 124) agissant par compression mécanique de l'emballage (116, 118).

9. Appareil selon la revendication 1, le pressuriseur étant une pompe.

10. Appareil selon la revendication 1, l'unité portable (110) étant prévue pour contenir deux emballages (116, 118) contenant du liquide.

11. Appareil selon la revendication 1, l'unité portable (110) étant prévue pour contenir deux emballages (116, 118) contenant du liquide et le pressuriseur (122, 124) étant prévu pour être en communication de pression avec les deux emballages (116, 118).

12. Appareil selon la revendication 1, l'unité portable (110) étant prévue pour contenir deux emballages (116, 118) contenant du liquide, et comportant deux pressuriseurs (122, 124), chaque pressuriseur (122, 124) étant prévu pour être en communication de pression avec un emballage (116, 118) distinct.

13. Appareil selon la revendication 1, comportant en outre la conduite servant à administrer le liquide au sujet.

14. Appareil selon la revendication 13, la conduite comportant en outre un éliminateur d'air incorporé dans la conduite.

15. Appareil selon la revendication 1, le moyen (144) de refroidissement étant configuré pour refroidir le liquide dans l'emballage (116, 118) contenant du liquide jusqu'à une température comprise entre environ 0°C et 10°C.

16. Appareil selon la revendication 1, le moyen de refroidissement comprenant une plaque froide (144).

17. Appareil selon la revendication 1, l'unité (120) de base fixe comportant en outre un capteur de température et une commande servant à surveiller et à réguler la température du moyen de refroidissement.
